# FASCICULE DE BREVET EUROPEEN

(11) **EP 0 782 009 B1**
(45) Date de publication et mention de la délivrance du brevet: **10.06.1998**
(21) Numéro de dépôt: 95402819.7
(22) Date de dépôt: 14.12.1995
(51) Int. Cl.: G01T 1/164, G01T 1/29

(54) **Procédé d'acquisition en médecine nucléaire d'une image en transmission**
Verfahren zur Erzeugung eines Übertragungsbildes in Nuklearmedizin
Method of obtaining transmission image in nuclear medicine

(43) Date de publication de la demande: 02.07.1997
(73) Titulaire: SMV INTERNATIONAL, 78534 Buc Cedex (FR)
(72) Inventeur: Bourguignon, Michel, F-75116 Paris (FR); Pare, Christian, F-75116 Paris (FR)
(74) Mandataire: Schmit, Christian Norbert Marie

(56) Documents cités:
- EP-A- 0 106 267
- EP-A- 0 526 970
- WO-A-91/02265
- FR-A- 2 721 411
- THE JOURNAL OF NUCLEAR MEDICINE, vol. 29, no. 2, Février 1988, NEW YORK,USA, pages 203-207, XP002004009 MACEY,D J ET AL.: "comparison of three boundary detection methods for spect using compton scattered photons"

## Description

La présente invention a pour objet un procédé d'acquisition, en médecine nucléaire, d'une image en transmission du corps d'un patient sous examen. L'image en transmission ainsi obtenue peut être utilisée telle quelle pour révéler l'anatomie du corps examiné. De préférence, elle sera utilisée pour corriger les résultats d'une image en émission révélée par la même machine. L'intérêt de l'invention est que dans ce dernier cas il est possible d'acquérir simultanément les deux images, d'où un gain de temps, et d'où surtout une bien meilleure comparaison géométrique des deux images acquises pour pouvoir corriger les résultats de l'une en fonction de ceux de l'autre.

La médecine nucléaire est une discipline du diagnostic médical dans laquelle on cherche à obtenir des informations fonctionnelles du corps des patients examinés. Dans ce but, on injecte dans ces patients des marqueurs radioactifs, généralement il s'agit de Technétium. Ce marqueur radioactif, en fonction de l'agent biologique qui le véhicule, va se fixer dans un organe ou dans un autre du patient. Là où il est fixé il émet des rayonnements radio-actifs: des rayons gamma, dont le nombre est d'autant plus grand qu'une plus grande quantité de marqueurs s'est fixée dans l'organe. Il s'agit là d'un phénomène d'émission.

Pour mesurer ce phénomène radio-actif, on utilise une gamma-caméra, ou caméra à scintillation. Une machine de médecine nucléaire comporte donc essentiellement un statif pour supporter une telle gamma-caméra. Celle-ci comporte un détecteur et des moyens de calculs de l'image détectée. Le détecteur comporte un cristal scintillateur. En général le cristal scintillateur est plan. Le scintillateur absorbe le rayonnement radio-actif gamma. Par effet photo-électrique, il émet en réponse une scintillation lumineuse, détectée en aval par un réseau de tubes photo-multiplicateurs du détecteur. Une gamma-caméra comporte de plus une console de commande et de traitement. Les tubes sont ainsi associés à des moyens de calcul. Ceux-ci permettent de déterminer les coordonnées d'un lieu d'interaction des rayonnements gamma dans le scintillateur. Ce lieu est révélateur de l'image en projection du corps.

Compte-tenu de ce que les émissions radio-actives dans le corps sont omnidirectionnelles, cette localisation ne peut être menée à bien qu'en interposant entre le corps et le scintillateur un collimateur. Ce collimateur ne laisse passer que les rayonnements radio-actifs se propageant dans une direction choisie.

Avec de tels examens en émission, on est capable de produire des images en projection. Si on fait tourner la gamma-caméra autour du corps du patient pendant que le phénomène radio-actif se produit (pendant une durée d'examen d'environ une demi-heure), on est susceptible d'acquérir un certain nombre de projections, par exemple de type parallèle, avec lesquelles selon les procédés de tomographie de type connu on sait reconstruire des images de coupe du corps. Les projections sont de type parallèle parce que le collimateur ne laisse passer les rayons que dans une seule direction perpendiculaire à son plan.

Le mode d'acquisition ainsi brièvement rappelé présente cependant un inconvénient: les rayons gamma émis par les structures internes du corps doivent, avant d'aller exciter le scintillateur, traverser d'autres régions de ce corps et ils y subissent une atténuation correspondante. Celle-ci perturbe l'acquisition des images acquises, et l'exactitude et la précision des images reconstruites. De nombreuses tentatives ont été faites pour prendre en compte cette atténuation, quelques fois sans la mesurer vraiment. Cependant, les méthodes ainsi proposées se sont révélées peu fructueuses, et à ce jour la mesure réelle de l'atténuation, notamment par tomographie de transmission, est la seule solution qui paraisse envisageable. Connaissant par une telle méthode, en chaque endroit du corps, le coefficient d'atténuation lié à la densité radiologique, on peut corriger les résultats des images d'émission. On tient compte pour la correction de la masse plus ou moins grande des tissus qui ont été interposés sur le chemin d'une émission radio-active issue d'une désintégration du marqueur.

Dans une demande de brevet français N° 89 10225 déposée le 28 Juillet 1989 et publiée sous le numéro 2 650 397 on a préconisé une telle mesure par transmission des structures internes du corps avec un dispositif comportant, face au détecteur de la gamma-caméra, une source ponctuelle externe de rayonnement gamma. Le corps est interposé entre cette source et ce détecteur. En pratique on a montré qu'il pouvait suffire d'éloigner cette source ponctuelle d'environ un mètre de la face du détecteur pour obtenir des images en transmission. Le terme en transmission signifie ici que le rayonnement mesuré est un rayonnement qui traverse le corps de part en part, la source de rayonnements étant externe: le corps transmet (ou ne transmet pas) le rayonnement gamma. Pour la reconstruction des images de tomographie, dans cette demande de brevet, on a montré qu'il suffisait de corriger légèrement les algorithmes de reconstruction pour tenir compte de la nature conique du rayonnement.

Cependant cette méthode présente des inconvénients. Le premier inconvénient est qu'il faut soumettre le corps du patient à une première irradiation avec cette source externe et il y a donc une perte de temps. Le deuxième inconvénient vient de la nature conique du rayonnement. En effet, du fait des tailles limitées du détecteur, la totalité du corps du patient ne peut être comprise dans le cône de radiation. Certaines des parties du corps, proches des génératrices du cône ne sont utilement soumises aux radiations que lorsque ces parties se trouvent les plus proches du plan du détecteur. Pour des raisons d'acquisition d'une image tomographique, la gamma-caméra a tourné d'un demi-tour autour du corps du patient. Ces mêmes régions sont alors les plus éloignées du plan du détecteur et soit ne sont pas irradiées, soit sont irradiées mais le résultat de cette irradiation déborde des limites du détecteur.

En conséquence la reconstruction des images, pour ces parties qui sont quelques fois prises en compte et quelques fois pas prises en compte conduit à des artefacts. En pratique on doit se limiter pour la reconstruction à la partie qui est vue quelle que soit l'incidence de la gamma-caméra.

Par ailleurs, dans un article intitulé: "Comparison of three boundary detection methods for SPECT using Compton scattered photons" dû à Messieurs D. J. MACEY et al., publié en février 1988 dans le volume 29 N° 2 du Journal of Nuclear Medicine, il a été imaginé d'acquérir des images de transmission par utilisation de l'effet Compton. Dans ce but, cet article se livre à une comparaison des images obtenues selon que la source radio-active est à l'intérieur ou à l'extérieur du corps du patient, et, dans ce dernier cas, selon qu'on est amené à mesurer des photons Compton à 90° ou à 180°. Le marqueur radio-actif utilisé est du Technétium dont l'énergie de radiation est de 140 Kev. Dans tous les cas des séries d'expérimentations sont entreprises en faisant tourner le corps devant le détecteur de la gamma-caméra.

Les conclusions de cet article sont que les résultats de l'expérimentation avec source radio-active interne sont inutilisables, alors que ces résultats sont bons lorsque la source est externe. Dans tous les cas il ne s'agit pas d'une transmission vraie mais d'une pseudo-transmission: tout simplement les rayonnements ne viennent pas directement d'un organe injecté. On utilisera néanmoins dans la suite de cet exposé le terme de transmission pour l'opposer à émission (qui est liée à l'injection du marqueur).

Pour l'expérience dans laquelle le marqueur radio-actif a été injecté dans la structure sous examen, la fenêtre d'énergie ouverte pour mesurer les photons Compton était centrée sur 110 Kev et avait une largeur de plus ou moins 15%: de 94 à 127 Kev. En pratique, compte tenu que pour le Technétium à 140 Kev, la limite inférieure de l'énergie des photons Compton pris en compte est de 90 Kev (rétrodiffusion à 180°) et que par ailleurs, pour des raisons de détection, il est admis d'ouvrir une fenêtre de plus ou moins 10% autour de 140 Kev (126 Kev - 154 Kev) pour mesurer le rayonnement gamma primaire, on peut considérer que cette expérimentation a pris en compte tous les photons Compton possibles. Néanmoins, les conclusions tirées par les auteurs de l'article font que la reconstruction de contours avec une source interne est difficile, et même n'est pas assez efficace pour effectuer une appréciation quantitative.

Le problème est donc de pouvoir se passer d'une source externe, dont la manipulation est par ailleurs toujours dangereuse pour les manipulateurs, et qui est la raison de pertes de temps et donc de rentabilité de la machine.

L'idée de l'invention est cependant d'utiliser la source de rayonnements gamma, telle qu'elle est injectée dans le patient, pour acquérir une image de transmission, en même temps qu'on acquiert l'image d'émission due au marqueur radio-actif. Dans ce but, dans l'invention, plutôt que d'ouvrir une fenêtre d'énergie tendant à prendre en compte les photons Compton primaires, qui à priori sont les plus nombreux, on a choisi au contraire de prendre en compte les photons Compton secondaires, voire même tertiaires, en abaissant la fenêtre d'énergie de façon qu'une partie notable de celle-ci soit située au delà de la limite inférieure d'émission des photons Compton primaires. Par exemple, pour des photons Compton primaires produits par du Technétium cette limite est de 90 Kev.

En pratique, on s'est rendu compte, que d'une part en agissant de cette façon on obtenait suffisamment d'informations pour pouvoir produire des images, et que d'autre part, les images ainsi produites n'étaient pas affectées d'artefacts. Il est vraisemblable qu'il se passe, avec le choix de l'invention, une meilleure prise en compte des différentes parties du corps, du fait que les photons Compton secondaires ou tertiaires ont des lieux d'émission, lieux où se produisent les événements Compton secondaires ou tertiaires, mieux répartis à l'intérieur du corps.

On a fait divers essais et on a pu constater que dès que la gamme prise en compte comportait une part notable située hors de celle du rayonnement primaire et de celle des photons Compton primaire, on était capable d'obtenir une bonne image.

L'invention a donc pour objet un procédé d'acquisition en médecine nucléaire, d'une image en transmission du corps d'un patient caractérisé en ce qu'il comporte les étapes suivantes:
- on place le corps du patient dans une machine de médecine nucléaire,
- on injecte dans le corps du patient un marqueur irradiant émettant des rayonnements radioactifs dans une gamme d'énergie donnée E,
- on détecte avec un détecteur de la machine des rayonnements se produisant à l'intérieur du corps du patient et dont la gamme d'énergie est comprise entre 0,75 E et 0,35 E.

Selon un explication de l'invention, on injecte dans le corps du patient un marqueur irradiant émettant des rayonnements radioactifs dans une gamme d'énergie donnée E, et on détecte avec un détecteur de la machine des rayonnements se produisant à l'intérieur du corps du patient et dont l'énergie correspond pour l'essentiel à celle de la gamme des réémissions de type Compton au moins secondaires ou tertiaires, c'est à dire dont la limite supérieure est fixée par la réémission à 180°.

Selon une autre explication de l'invention on détecte avec un détecteur de la machine des rayonnements se produisant à l'intérieur du corps du patient dont la gamme d'énergie est telle que les lieux de ces réémissions sont répartis statistiquement sensiblement également dans tous les endroits.

L'invention sera mieux comprise à la lecture de la description qui suit et à l'examen des figures qui l'accompagnent. Celles-ci ne sont données qu'à titre indicatif et nullement limitatif de l'invention. Les figures montrent:
Figures 1 et 2: Des vues de face et en coupe d'une machine de médecine nucléaire utilisable pour mettre en oeuvre le procédé de l'invention;
Figure 3: La représentation schématique du fonctionnement d'un détecteur d'une gamma-caméra;
Figure 4: Un diagramme d'énergie d'un événement radio-actif et les plages d'énergie préférées du procédé de l'invention;
Figure 5: Une représentation schématique d'un rayonnement Compton secondaire ou tertiaire.

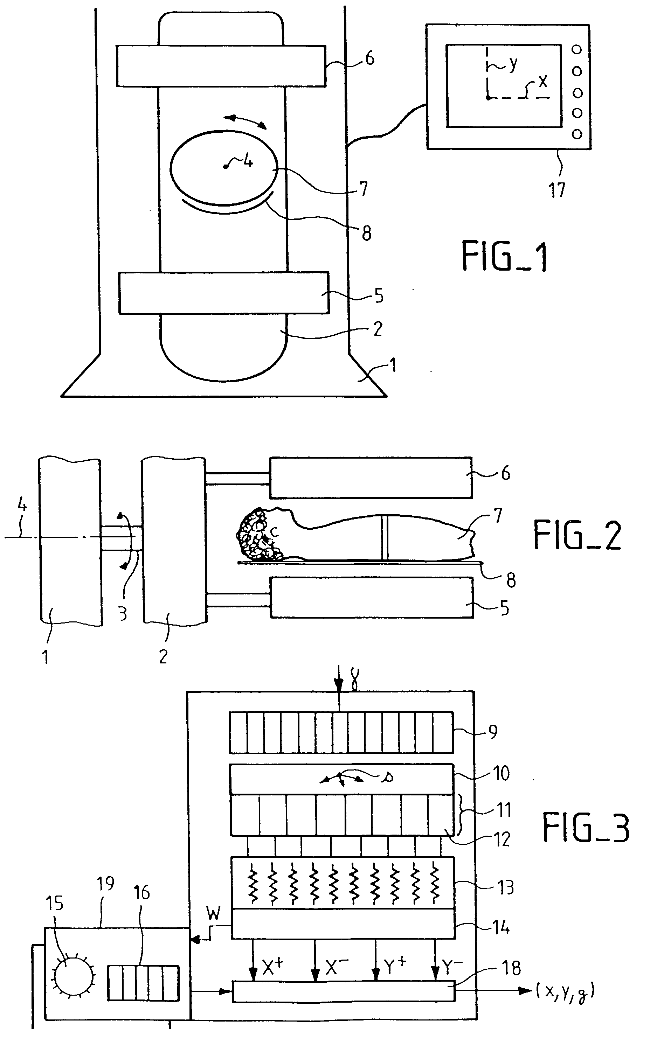

Les figures 1 et 2 montrent une machine de médecine nucléaire, dite aussi gamma-caméra, utilisable pour mettre en oeuvre le procédé de l'invention. La figure 1 est une vue de face, la figure 2 est une coupe de la vue de la figure 1 selon un plan vertical perpendiculaire à cette figure 1. La machine comporte un bâti 1 qui maintient un statif 2. Le statif 2 est maintenu sur le bâti 1 par un arbre de rotation 3 pouvant tourner, dans le sens des flèches autour d'un axe 4. Le statif 2 maintient, dans cet exemple, deux détecteurs 5 et 6 écartés l'un de l'autre pour recevoir entre eux le corps 7 d'un patient posé sur un lit 8. La machine pourrait comporter néanmoins un seul détecteur, ou plus que deux. On connaît des machines possédant ainsi jusqu'à une vingtaine de détecteurs.

Le principe de l'acquisition d'une image est le suivant. Pour une position donnée en orientation des détecteurs par rapport au corps 7, les rayons gammas issus de ce corps traversent un collimateur 9 (figure 3) placé sur une face du détecteur en regard du corps 7. Le collimateur 9 peut être à trous droits ou obliques, parallèles ou convergents. La présence d'un collimateur permet d'éviter la prise en compte d'un rayonnement diffusé gênant. Après passage dans le collimateur, les photons gamma qui ont la bonne direction de propagation viennent provoquer une scintillation s dans un cristal scintillateur 10 placé de l'autre côté du collimateur 9. La scintillation s produite par le scintillateur 10 est détectée par un réseau 11 de tubes photo-multiplicateurs tels que 12 qui produisent des signaux électriques correspondant à la scintillation qu'ils ont détectée. Ces signaux sont transmis à des circuits de traitement comportant notamment un réseau 13 de résistance de barycentration pour pondérer les signaux électriques produits par les différents tubes afin d'élaborer des signaux électriques de localisation dits X+, X-, Y+ et Y-représentatifs d'un lieu dans le scintillateur 10 où s'est produite la scintillation mesurée. Dans ce but le réseau des résistances 13 est relié à un circuit d'amplification 14 qui élabore ces signaux électriques de localisation.

Le circuit 14 produit également un signal d'énergie W permettant, pour chaque scintillation s de connaître son énergie totale.

Au moment d'une expérimentation, la gamme d'énergie dans laquelle est destinée à fonctionner la machine est réglée au moyen de boutons de commande tels que 15 et d'indicateurs de réglage 16. En pratique les boutons 15 peuvent tout simplement être un clavier d'un ordinateur de commande de la machine, et l'afficheur 16 l'écran 17 de cet ordinateur. Ce faisant on dispose classiquement, sur n'importe quelle machine de médecine nucléaire, des moyens d'indiquer quelle est la gamme d'énergie à prendre en compte. Par exemple pour des images en émission avec un marqueur radio-actif de type Technétium émettant des photons gamma à 140 Kev, il est classique de centrer la fenêtre d'énergie de réception sur 140 Kev et de l'ouvrir de plus ou moins 10%: entre 154 Kev et 126 Kev.

Le dispositif 19 de réglage de cette gamme d'énergie est couplé à un circuit 18 de filtrage qui valide la prise en compte, dans la suite du traitement effectué par la gamma-caméra, des signaux de localisation si l'énergie de l'impulsion gamma mesurée se trouve dans la gamme choisie.

Dans l'invention on règle au premier chef, avec les boutons 15, la gamme pour qu'elle soit située entre 0,75 E et 0,35 E, E étant l'énergie des rayons gamma émis normalement par le marqueur radio-actif utilisé. Le réglage de cette gamme doit en pratique correspondre pour l'essentiel à la gamme des réémissions de type Compton au moins secondaires ou tertiaires. On pense qu'en agissant de cette façon là, on est sûr d'obtenir une répartition statistique égale des lieux de réémission. En effet, on peut estimer que la densité des réémissions Compton primaires est décroissante au fur et à mesure qu'on s'éloigne de la source de rayons gamma, qu'on s'éloigne de l'organe qui a été injecté dans le corps. Par contre s'agissant des rayonnements secondaires ou primaires, il semblerait que leur répartition soit meilleure. En tout cas cela conduit, comme on a pu le constater par l'expérience, à un meilleur rendu des images. Notamment les contours qui n'apparaissent pas dans l'article cité sont maintenant bien révélés.

Chaque détecteur comporte donc en aval du circuit 14 un circuit 18 qui a deux missions. D'une part, il produit des signaux électriques X et Y qui renseignent sur la position exacte dans l'image produite du lieu de la scintillation s (et donc en correspondance du lieu, en projection, où s'est produite l'émission gamma primaire ou le rayonnement Compton primaire, secondaire, tertiaire dans le corps 7). La détection de cet événement se traduit par la révélation sur l'écran 17 de visualisation des images d'un point lumineux à l'endroit de l'image qui corresponde à ces coordonnées X,Y. Cependant cette révélation ne se fait d'autre part que si par ailleurs l'énergie W mesurée correspond à la gamme réglée avec les boutons 15. Si l'énergie mesurée correspond à cette gamme, le circuit 19 de sélection de fenêtre délivre un signal qui valide le fonctionnement du circuit 18. Dans le cas contraire celui-ci ne produit rien: l'événement radio-actif est ignoré.

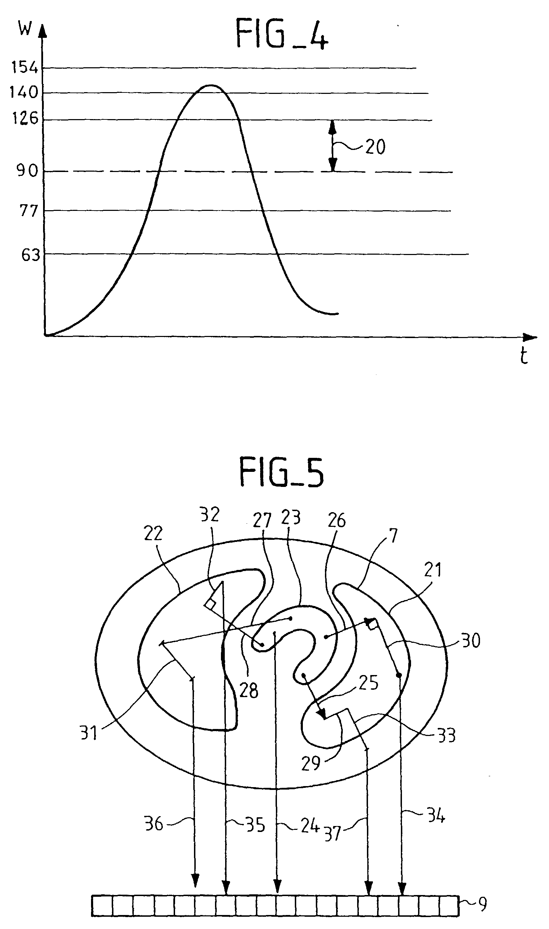

La figure 4: montre un signal d'énergie W évoluant en fonction du temps au moment où se produit une scintillation. La durée de l'impulsion électrique délivrée par les tubes 12 pour chaque scintillation est très courte: de l'ordre de quelques nanosecondes. Pendant cette durée le signal électrique croît puis décroît pour revenir à zéro. S'il s'agit d'un rayonnement gamma primaire dû au Technétium cette impulsion doit normalement culminer avec une valeur correspondant à 140 Kev. Aussi, pour révéler des images en émission (où on prend en compte le rayonnement gamma primaire) on règle la fenêtre de détection entre 126 Kev et 154 Kev. Le rayonnement Compton primaire, pour un même marqueur, produit des rayons gamma dont l'énergie n'est pas inférieure à 90 Kev. La partie de la gamme de la figure 4 marquée par la double flèche 20 était la partie d'énergie scrutée par les expérimentations décrites dans l'article cité ci-dessus et qui ont conduit au rejet de la technique.

Par rapport à cet enseignement négatif, l'invention apporte que , au contraire, on obtient de très bons résultats à partir du moment où on centre la fenêtre de détection au delà de la valeur limite basse de production des rayonnements Compton primaires.

On a pu observer avec l'invention qu'on obtenait des bons résultats, avec des fenêtres à plus ou moins 15%, dès que le centre de cette fenêtre était placé en deçà de l'énergie minimale des rayonnements Compton primaires. Par exemple pour le Technétium on peut choisir un centre de fenêtre jusqu'à 90 Kev et donc une fenêtre ouverte entre 105 Kev et 75 Kev. De même des valeurs assez basses ont été testées qui donnent également de bons résultats. En pratique, on a pu déterminer que la gamme d'énergie à utiliser devait être comprise entre 0,75 x E et 0,35 x E, E, étant l'énergie de rayonnement gamma de le marqueur marqueur utilisé.

Les gamma-caméras de l'état de la technique sont normalement conçues pour pouvoir régler avec le dispositif 19 une fenêtre correspondant au Technétium: 140 Kev. Elles sont par ailleurs aussi conçues pour régler la réception sur une fenêtre d'énergie correspondant au Thallium: 70 Kev. En pratique dans l'invention, on choisira, puisqu'alors les protocoles d'acquisition relatifs au Thallium sont normalement préprogrammés dans toutes les machines, de mettre en oeuvre, pour révéler l'image de transmission, le protocole du Thallium alors que le marqueur injecté dans le corps est du Technétium. De ce fait il n'y a rien à changer dans les machines existantes pour leur faire révéler l'image de transmission acquise selon le procédé de l'invention. Il suffit simplement de choisir une gamme d'énergie comme on l'a indiqué plus haut.

Quand on veut acquérir cependant les deux images, émission et transmission simultanément, on choisit de doubler le circuit 19 soit physiquement et dans ce cas on doublera également le circuit 18, soit, de préférence, on modifie le programme de traitement mis en oeuvre par l'ordinateur de traitement d'images utilisé par la machine. Classiquement, une machine de médecine nucléaire comptabilise, pour une durée donnée, en chaque point d'image d'une image à créer, le nombre des événements radioactifs qui s'y sont produits. La luminosité des points de l'image est fonction de ce nombre. Cette répartition des luminosités révèle les contours de l'image du corps. Donc normalement le système de traitement d'images de la machine comporte un programme d'ordinateur qui incrémente d'une unité le nombre des événements affectés à un lieu X, Y dans l'image à chaque fois qu'il reçoit des signaux X, Y délivrés par le circuit 18. Dans l'invention les signaux délivrés par le circuit 18, soit seront dupliqués parce qu'il y aura deux circuits 18 , soit de préférence seront complétés par une information g relative à la gamme et qui sera donnée par le ou les circuits 19.

Ainsi lorsqu'un événement radio-actif se produit dans le corps et est détecté par le scintillateur, soit cet événement correspond à un rayonnement primaire (140 Kev) et la comptabilisation de l'événement est affectée à l'image d'émission (par exemple g = zéro). Soit l'énergie de cet événement radio-actif a culminé dans la gamme sélectionnée pour les événements Compton secondaires ou tertiaires (70 Kev plus ou moins 10% = 77 Kev-63Kev) et l'événement est comptabilisé aux coordonnées X,Y pour contribuer à construire l'image de transmission (et dans ce cas g = 1). Soit encore l'énergie de l'événement radio-actif a culminé en dehors des gammes sélectionnées. Dans ce cas il est tout simplement éliminé et les signaux électrique X et Y ne sont transmis à aucune des deux images. A l'issue, on obtient les deux images simultanément.

La figure 5 montre une représentation schématique de ce qui se produit à l'intérieur du corps 7 du patient pendant toute la durée de l'acquisition. On distingue sur cette figure les poumons 21 et 22 ainsi que le coeur 23 représenté en coupe. Du fait de l'agent biologique utilisé, on peut estimer que seul le coeur 23, au moins pendant un premier temps, est émetteur de rayonnement gamma primaire. Des rayonnements gamma primaires 24 vont ainsi attaquer le collimateur 9. S'ils ont la bonne orientation il traversent le collimateur et sont pris en compte pour révéler la présence du coeur dans l'image d'émission.

Parmi les rayonnements gamma qui ne sont pas pris en compte pour l'image d'émission, il y a des rayonnements gamma qui subissent des diffusions Compton telles que représentés en 25 à 28. A l'endroit de l'impact Compton, il y a production d'un rayonnement Compton primaire, respectivement 29 à 32. Ces photons Compton primaires donnent eux-mêmes naissance à des photons Compton secondaires 33 à 36 dont certains sont détectés par le détecteur 5 parce qu'ils ont la bonne orientation: les photons 34 à 36 et certains ne le sont pas, soit parce qu'ils n'ont pas la bonne orientation soit parce qu'ils donnent eux-mêmes naissance à un photon Compton tertiaire 37 qui lui aura la bonne orientation. Il est vraisemblable qu'avec le choix de l'invention d'utiliser des rayonnements Compton secondaires ou tertiaires, on obtient une meilleure répartition dans le corps 7 des lieux de naissance de ces photons Compton de bas niveau d'énergie: ces lieux sont répartis plus uniformément que ne pourraient l'être les rayonnements Compton primaires.

On obtient ainsi un résultat étonnant en ce sens que pour révéler le fonctionnement du coeur on a pu continuer à concentrer avec l'agent biologique retenu la présence du marqueur radio-actif dans le coeur tout en obtenant par ailleurs une bonne répartition, à travers tous le corps du patient, des lieux d'émission des rayonnements Compton secondaires ou tertiaires. On est, en pratique, pas obligé d'injecter le patient avec un marqueur qui se répandrait partout dans le corps de ce patient. Finalement, une des difficultés mises en évidence par l'article cité ci-dessus et qui était tourné en extrayant la source radio-active du corps, est maintenant résolue en maintenant la source radio-active à l'intérieur du corps (dans le coeur), mais en obtenant une meilleure répartition des lieux de création des photons à détecter en choisissant une gamme d'énergie plus faible.

Pour la correction de l'image d'émission, qu'on obtient simultanément à l'image de transmission, on a découvert de plus qu'il suffisait de se satisfaire d'une image de transmission segmentée et quantifiée sur un nombre de segments limité. Dans le cas d'une segmentation binaire, par exemple, on considère ainsi que les tissus sont denses ou pas denses, selon que le nombre de coups comptés à l'aplomb de chaque endroit de l'image est supérieur ou inférieur à un seuil. Cette binarisation de l'image peut être effectuée de deux façons. Soit, de préférence, on acquiert pour plusieurs incidences des détecteurs autour du corps du patient, des images de transmission, et avec ces images en projection sous différentes incidences on reconstruit, comme pour une image d'émission normale, une image de transmission. Cette image de transmission est alors rendue binaire ou ternaire ou quaternaire... On corrige ensuite selon les modes connus l'image d'émission en fonction de cette image binaire ou de pseudo-projection qu'on en déduit. On pourrait par ailleurs utiliser une binarisation des images de transmission en projection obtenues pour chacune des incidences avant de recalculer la tomographie de l'image d'émission dont toutes les projections seraient corrigées. Pour la correction on attribue à chaque élément d'image segmentée un coefficient d'atténuation correspondant aux tissus biologiques identifiés. Cette correspondance est de préférence à priori. Par exemple elle est 0,15 cm ⁻¹ pour les tissus biologiques, de 0,18 cm⁻¹ pour le sang, 0,4 cm⁻¹ pour les poumons.

On peut injecter le marqueur de manière à ce qu'il se fixe dans le coeur. On peut également l'injecter sous une autre forme de manière à ce qu'il se fixe dans le foie, dans les poumons etc... Dans tous les cas on choisira de préférence une fixation localisée si on veut acquérir en même temps les deux images.

## Revendications

1. Procédé d'acquisition, en médecine nucléaire, d'une image en transmission du corps (7) d'un patient comportant les étapes suivantes selon lesquelles :
- on place le corps (7) du patient dans une machine de médecine nucléaire ;
- on injecte, dans le corps (7) de ce patient, un marqueur irradiant émettant des rayonnements radioactifs dans une gamme d'énergie donnée E ; et caractérisé en ce qu'il comporte en outre l'étape suivante selon laquelle :
- on détecte, avec un détecteur (5, 6) de ladite machine de médecine nucléaire, des rayonnements se produisant à l'intérieur du corps (7) du patient et dont la gamme d'énergie est comprise entre 0,75 E et 0,35 E.

2. Procédé d'acquisition, en médecine nucléaire, d'une image en transmission du corps (7) d'un patient comportant les étapes suivantes selon lesquelles :
- on place le corps (7) du patient dans une machine de médecine nucléaire ;
- on injecte, dans le corps (7) de ce patient, un marqueur irradiant émettant des rayonnements radioactifs dans une gamme d'énergie donnée E ;
et caractérisé en ce qu'il comporte en outre l'étape suivante selon laquelle :
- on détecte, avec un détecteur (5, 6) de ladite machine de médecine nucléaire, des rayonnements se produisant à l'intérieur du corps (7) du patient et dont l'énergie correspond pour l'essentiel à celle de la gamme des réémissions de type Compton au moins secondaires (33, 34, 35, 36) ou tertiaires (37).

3. Procédé selon l'une des revendications 1 ou 2, caractérisé en ce que
- on détecte, avec un détecteur (5, 6) de la machine de médecine nucléaire, des rayonnements se produisant à l'intérieur du corps du patient et dont la gamme d'énergie est comprise entre 0.55 E et 0,45 E.

4. Procédé selon l'une des revendications 1 à 3, caractérisé en ce que
- on acquiert l'image de transmission en même temps qu'une image d'émission en mesurant dans les rayonnements émanant du corps (7) du patient ceux compris entre 0,35 E et E et en séparant ceux correspondant à l'énergie E pour produire l'image d'émission de ceux correspondant à la gamme d'énergie de transmission.

5. Procédé selon l'une des revendications 1 a 4, caractérisé en ce que
- on injecte un marqueur contenant du Technétium 99m, et on détecte, pour l'image de transmission, les rayonnements se produisant dans une gamme d'énergie correspondant au Thallium.

6. Procédé selon l'une des revendications 1 à 5, caractérisé en ce que
- au cours de l'acquisition on fait occuper au détecteur plusieurs positions autour du corps (7) du patient, et
- on en déduit une image tomographique de densité radiologique des tissus à l'intérieur de ce corps (7).

7. Procédé selon la revendication 6, caractérisé en ce que
- on segmente l'image tomographique de densité radiologique en attribuant à chaque élément d'image de cette image des densités d'un type donné selon que la densité mesurée pour ces éléments d'image est comprise dans une gamme.

8. Procédé selon l'une des revendications 6 ou 7, caractérisé en ce que
- on corrige l'image d'émission en fonction des résultats de l'image de transmission.

9. Procédé selon l'une des revendications 1 à 9, caractérisé en ce qu'on injecte le marqueur radioactif de telle façon que celui-ci se fixe dans le coeur (23) ou les poumons (21, 22) du patient.

## Patentansprüche

1. Verfahren zur nuklearmedizinischen Erzeugung eines auf der Transmission eines Körpers (7) beruhenden Bilds eines Patienten, umfassend die Schritte:
- Einbringen des Körpers (7) des Patienten in eine nuklearmedizinische Vorrichtung;
- Injizieren eines strahlenden Markers, der radioaktive Strahlen in einem vorbestimmten Energiebereich E emittiert, in den Körper (7) dieses Patienten; und dadurch gekennzeichnet, daß es ferner einen Schritt umfaßt, gemäß dem
- mit einer Erfassungseinrichtung (5, 6) dieser nuklearmedizinischen Vorrichtung Strahlung erfaßt wird, die im Innern des Körpers (7) des Patienten entsteht und deren Energiebereich zwischen 0,75 E und 0,35 E liegt.

2. Verfahren zur nuklearmedizinischen Erzeugung eines auf der Transmission eines Körpers (7) beruhenden Bilds eines Patienten, umfassend die Schritte:
- Einbringen des Körpers (7) des Patienten in eine nuklearmedizinische Vorrichtung;
- Injizieren eines strahlenden Markers, der radioaktive Strahlen in einem vorbestimmten Energiebereich E emittiert, in den Körper (7) dieses Patienten; und dadurch gekennzeichnet, daß es ferner einen Schritt erfaßt, gemäß dem
- mit einer Erfassungseinrichtung (5, 6) dieser nuklearmedizinischen Vorrichtung Strahlung erfaßt wird, die im Innern des Körpers (7) des Patienten entsteht und deren Energie im wesentlichen der des Bereichs der zumindest sekundären (33, 34, 35, 36) oder tertiären (37) Compton-Reemissionen entspricht.

3. Verfahren nach einem der Ansprüche 1 oder 2, dadurch gekennzeichnet, daß
- mit einer Erfassungsvorrichtung (5, 6) dieser nuklearmedizinischen Vorrichtung Strahlung erfaßt wird, die im Innern des Körpers des Patienten entsteht und deren Energiebereich zwischen 0,55 E und 0,45 E liegt.

4. Verfahren nach einem der Ansprüche 1 bis 3, dadurch gekennzeichnet, daß
- das Transmissionsbild gleichzeitig mit einem Sendebild erzeugt wird, indem in der aus dem Körper (7) des Patienten austretenden Strahlung diejenige, die zwischen 0,35 E und E liegt, gemessen wird, und indem diejenige, die der Energie E entspricht, abgetrennt wird, um das Strahlungsbild derjenigen, die dem Sendeenergiebereich entspricht, zu erzeugen.

5. Verfahren nach einem der Ansprüche 1 bis 4, dadurch gekennzeichnet, daß
- ein Technetium 99m enthaltender Marker injiziert wird und zum Erhalt des Transmissionsbilds die Strahlung erfaßt wird, die in einem Thallium entsprechenden Energiebereich entsteht.

6. Verfahren nach einem der Ansprüche 1 bis 5, dadurch gekennzeichnet, daß
- während der Erzeugung die Erfassungseinrichtung mehrere um den Körper (7) des Patienten liegende Positionen einnimmt, und
- ein tomographisches Bild der radiologischen Dichte von Gewebe im Innern dieses Körpers (7) abgeleitet wird.

7. Verfahren nach Anspruch 6, dadurch gekennzeichnet, daß
- das tomographische Bild der radiologischen Dichte segmentiert wird, indem je nachdem, wie die für diese Elemente gemessene Dichte von einem Bereich umfaßt wird, jedem Bildelement dieses Bilds Dichten eines gegebenen Typs zugewiesen werden.

8. Verfahren nach einem der Ansprüche 6 oder 7, dadurch gekennzeichnet, daß
- das Sendebild in Abhängigkeit von den Ergebnissen des Transmissionsbilds korrigiert wird.

9. Verfahren nach einem der Ansprüche 1 bis 9, dadurch gekennzeichnet, daß der radioaktive Marker derart injiziert wird, daß er sich im Herzen (23) oder in der Lunge (21, 22) des Patienten festsetzt.

## Claims

1. A method used in radionuclide medicine of acquiring an image transmitting the body (7) of a patient, consisting of the following steps whereby :
- the body (7) of the patient is placed in a radionuclide imaging machine;
- an irradiating marker which emits radioactive radiation within a given energy range E is injected into the body (7) of this patient; and characterised in that it additionally incorporates the following step whereby:
- a detector (5, 6) of said radionuclide imaging machine is used to detect rays produced inside the body (7) of the patient, the energy range of which is between 0.75 E and 0.35 E.

2. A method used in radionuclide medicine of acquiring an image transmitting the body (7) of a patient, consisting of the following steps whereby :
- the body (7) of the patient is placed in a radionuclide imaging machine;
- an irradiating marker emitting radioactive radiation within a given energy range E is injected into the body (7) of this patient;
and characterised in that it additionally incorporates the following step whereby :
- a detector (5, 6) of said radionuclide imaging machine detects rays produced inside the body (7) of the patient, the energy of which essentially corresponds to that of the range of at least secondary (33, 34, 35, 36) or tertiary (37) re-emissions of the Compton type.

3. A method as claimed in one of claims 1 or 2, characterised in that
- a detector (5, 6) of said radionuclide imaging machine detects rays produced inside the body of the patient, the energy range of which is between 0.55 E and 0.45 E.

4. A method as claimed in one of claims 1 to 3, characterised in that
- the transmission image is acquired at the same time as an emission image by measuring in the rays emanating from the body (7) of the patient those ranging between 0.35 E and E and by separating those corresponding to the energy E in order to produce the emission image of those corresponding to the transmission energy range.

5. A method as claimed in one of claims 1 to 4, characterised in that
- a marker containing technetium 99m is injected and the rays produced within an energy range corresponding to thallium are detected for the transmission image.

6. A method as claimed in one of claims 1 to 5, characterised in that
- during the acquisition process, the detector is moved to several positions around the body (7) of the patient and
- a tomographic image of radiological density is derived from the tissues inside this body (7).

7. A method as claimed in claim 6, characterised in that
- the tomographic image of radiological density is segmented by assigning densities of a given type to each image element of this image depending on the measured density for each of these image elements and contained within a range.

8. A method as claimed in one of claims 6 or 7, characterised in that
- the emission image is corrected on the basis of the transmission image results.

9. A method as claimed in one of claims 1 to 9, characterised in that the radioactive marker is injected so that it stays in the heart (23) or the lungs (21, 22) of the patient.
